# EUROPÄISCHE PATENTANMELDUNG

(11) **EP 1 703 269 A1**
(43) Veröffentlichungstag der Anmeldung: **20.09.2006**
(21) Anmeldenummer: 06405095.8
(22) Anmeldetag: 03.03.2006
(51) Int. Cl.: G01N 3/00, G01N 3/08

(54) **Einrichtung zum Testen von elastischen textilen Beinbekleidungen**

(30) Priorität: 14.03.2005 CH 4322005; 14.11.2005 CH 18132005
(71) Anmelder: Salzmann AG, 9001 St. Gallen (CH)
(72) Erfinder: Kuenzli, Daniel, 9053 Teufen (CH); Braun, Walter, 8735 St. Gallernkappel (CH); Ruettiger, Muck, 76473 Iffezheim (DE)
(74) Vertreter: Kulhavy, Sava

(57) **Zusammenfassung**

Die Einrichtung weist einen länglichen und vertikal verlaufenden Träger (90) für Drucksensoren (50) auf, welcher einer Gruppe von Hebeln (71) vorangestellt ist. Der Querschnitt dieses Trägers (90) ist bogenförmig, sodass er zwei Flanken (93,94) aufweist. Dieser Träger (90) simuliert während dem Testen einer Beinbekleidung (80) den Bereich des Schienbeins. Die Einrichtung weist ferner Formstücke bzw. Gegenlager (88) auf, welche durch die Hebel getragen sind und welche die rückwärtige Fläche der Wade bzw. des Oberschenkels eines Beines simulieren. Die Breite dieser Gegenlager entspricht der Breite der Wade bzw. des Oberschenkels eines Beines. Die Einrichtung umfasst auch zwei länglich verlaufende Gruppen (50) von Drucksensoren, von welchen je eine der Aussenseite einer der Flanken (93,94) des Trägers (90) zugeordnet ist. Die zu testende Beinbekleidung (80) wird zum Testen derselben über diese Einrichtung so überstülpt, dass sie auf den Aussenflächen sowohl des Trägers (90) als auch der Gegenlager aufliegt und auf die Drucksensoren (51) Druck ausübt. Zwischen dem Träger (90) und dem Gegenlager (88) erstrecken sich frei stehende Abschnitte (81,82) der Beinbekleidung (80). Es ist eine Auswertevorrichtung vorgesehen, welche die durch die Drucksensoren abgegebenen Werte auswerten und anzeigen kann.

## Beschreibung

Die vorliegende Erfindung betrifft eine Einrichtung zum Testen von elastischen textilen Beinbekleidungen, insbesondere von Strümpfen oder Strumpfhosen, von medizinischen Kompressions-Strümpfen und Stützstrümpfen.

Elastische textile Beinbekleidungen müssen so geformt sein, dass sie dem Umriss des Beins der die Beinbekleidung tragenden Person möglichst genau folgen. Denn nur in einem solchen Fall kann die Beinbekleidung ihre Funktion voll ausüben, ohne dass es Stellen entlang dem Bein gibt, an welchen die Beinbekleidung entweder einen zu grossen oder einen zu kleinen Druck ausübt. Um dies zu erreichen, ist es bekannt, das Bein des Trägers der Beinbekleidung zunächst zu vermessen. Diese Vermessung wird in der Weise durchgeführt, dass der Umfang des Beines an bestimmten Stellen des Beines gemessen wird. Bei dieser Vermessung misst man beispielsweise den Umfang des Beines im Bereich der Fessel, am Anfang, in der Mitte und am oberen Ende der Wade, im Kniebereich und im Bereich des Oberschenkels, nämlich in der Mitte und am oberen Ende desselben. Wenn es sich um eine Strumpfhose handelt, dann wird der Umfang auch im Bereich des Gesässes und gegebenenfalls auch im Bereich der Taille gemessen. Aufgrund dieser Vorgaben wird die Beinbekleidung dann hergestellt. Hiernach ist es allerdings auch erforderlich zu ermitteln, ob die Vorgaben beim hergestellten Produkt eingehalten worden sind.

Bereits sind Einrichtungen zur Vermessung fertiger Strümpfe bekannt. Bei einer dieser Einrichtungen werden die einander gegenüberliegenden Längsränder des flachgelegten Strumpfes auf Nadeln aufgesteckt, welche zwei Reihen entlang der Strumpflänge bilden. Diese Nadeln sind beweglich gelagert, und zwar derart, dass der Abstand zwischen zwei einander gegenüberliegenden Nadeln der Nadelreihen geändert werden kann. Die Messung, ob die Vorgaben während der Herstellung des Strumpfes eingehalten worden sind, kann beispielsweise in der Weise erfolgen, dass einander gegenüberliegende Nadeln in Abstände voneinander gebracht werden, welche den Vorgabe entsprechen und dass die Spannung im Strumpf zwischen zwei einander gegenüberliegenden Nadeln gemessen wird. So kann man zwar feststellen, ob die Kontur des Strumpfes den durch die Vorgaben bestimmten Verlauf aufweist. Wegen dem Aufspiessen auf die Nadeln wird der vermessene Strumpf jedoch unverkäuflich. Wenn man bedenkt, dass solche Strümpfe oft Einzelanfertigungen sind, dann ist die Vermessung der fertigen Strümpfe mit Hilfe der soeben genannten Einrichtung uneffektiv.

Die Aufgabe der vorliegenden Einrichtung ist, den genannten sowie noch weitere Nachteile des Standes der Technik zu beseitigen.

Diese Aufgabe wird bei der Einrichtung der eingangs genannten Gattung in der Weise gelöst, wie dies im kennzeichnenden Teil des Patentanspruchs 1 definiert ist.

Nachstehend werden Ausführungsformen der vorliegenden Erfindung anhand den beiliegenden Zeichnungen näher erläutert. Es zeigt:
Fig. 1 das Modell eines menschlichen Beines, an dem demonstriert werden kann, an welchen Stellen des Beines Umfangsmessungen durchgeführt werden können,
Fig. 2 in einer Seitenansicht eine erste Ausführungsform der vorliegenden Einrichtung, welche grösstenteils mit Hilfe eines kinematischen Schemas dargestellt ist,
Fig. 3 vergrössert einen Ausschnitt aus Fig. 2,
Fig. 4 einen bloss schematischen horizontalen Schnitt A-A durch eine weitere Ausführung der vorliegenden Erfindung, welche der Einrichtung gemäss Fig. 2 entspricht,
Fig. 5 eine noch weitere Ausführungsform der vorliegenden Einrichtung, deren
Darstellung weitgehend der Darstellung in Fig. 2 entspricht und
Fig. 6 in einer Draufsicht die in Fig. 5 dargestellte Ausführungsform der vorliegenden Einrichtung.

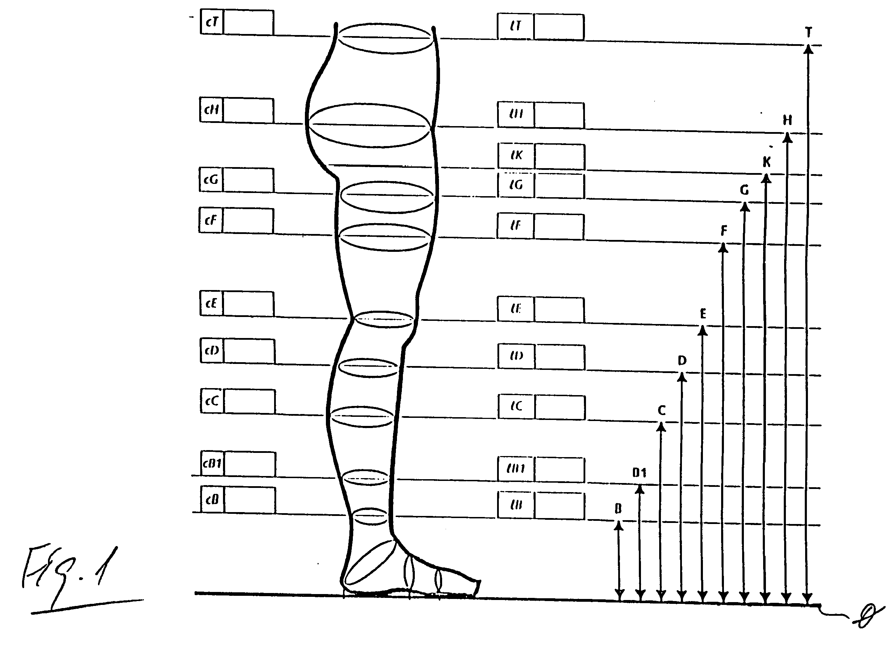

Fig. 1 zeigt das Modell eines menschlichen Beines, an dem demonstriert werden kann, an welchen Stellen bzw. in welchen Bereichen des menschlichen Körpers Umfangsmessungen durchgeführt werden können, um die Beinbeklei- ' dungen, wie z.B. medizinische Strümpfe oder Strumpfhosen anfertigen zu können. Im Prinzip könnte man auch den Fuss der Beinbekleidung vermessen. Für die Zwecke des vorliegenden Falles werden hier Messungen nur oberhalb des Fusses berücksichtigt. Während einer solchen Vermessung misst man beispielsweise den Umfang des Beines im Bereich der Fessel cB, am Anfang cB1 der Wade, in der Mitte cC der Wade und am oberen Ende cD der Wade, im Kniebereich cE, in der Mitte cF des Oberschenkels und am oberen Ende cG desselben. Wenn es sich um die Herstellung einer Strumpfhose handelt, dann wird auch der Umfang cH im Bereich des Gesässes und der Umfang cT der Taille vermessen.

Die menschlichen Beine unterscheiden sich voneinander nicht nur durch die Grösse des Umfanges in den genannten Bereichen desselben sondern auch durch die Abstände zwischen diesen Bereichen. Diese Abstandsunterschiede sind durch die unterschiedliche Länge der Beine bedingt. Deswegen werden auch diese Längen während der Vermessungen eines Beines ermittelt. Diese Längen werden zweckmässigerweise auf die Fussunterseite, insbesondere auf die Unterseite der Ferse bezogen. Der Bereich der Fessel cB befindet sich im Abstand LB von der Ferse usw., wie dies in Fig. 1 rechts angegeben ist.

Die vorliegende Einrichtung zum nicht zerstörenden Prüfen von elastischen textilen Beinbekleidungen, wie z.B. von Strümpfen, Strumpfhosen usw. ist so ausgeführt, dass sie erlaubt, die in den betreffenden Bereichen eines Beines gemessenen Umfangswerte in den entsprechenden Bereichen der Einrichtung einzustellen. Ferner ermöglicht die vorliegende Einrichtung, die Grösse der Spannung zu messen, welche in jenen Bereichen der Beinbekleidung herrschen, die den genannten Bereichen des Beines entsprechen. Dies garantiert dafür, dass das fertige Produkt nicht nur seine die Gesundheit unterstützende Wirkung ausübt sondern dass sich die Beinbekleidung auch komfortabel tragen lässt.

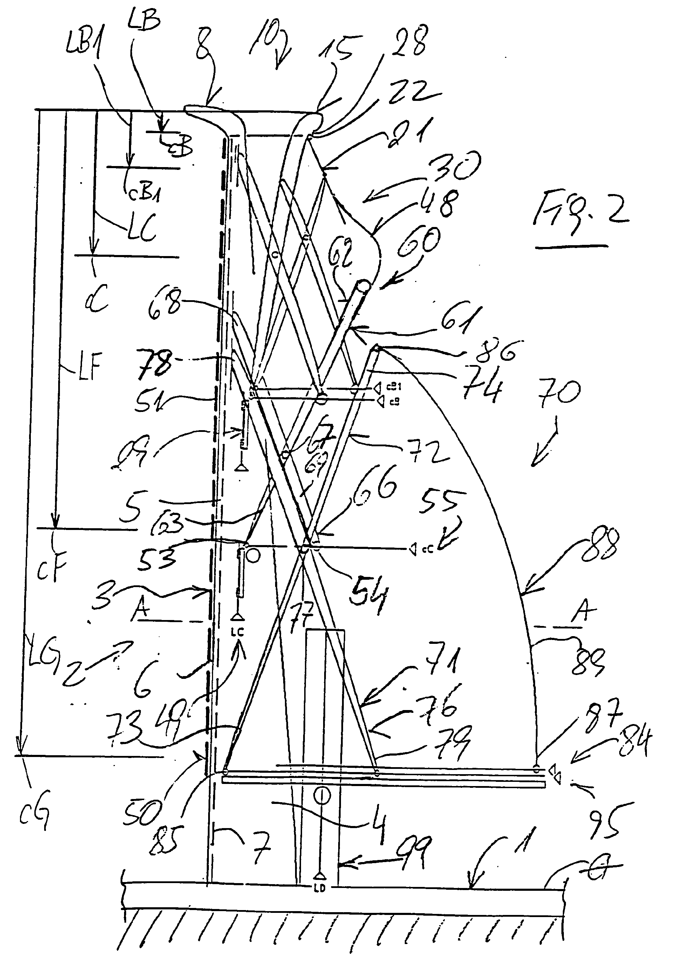

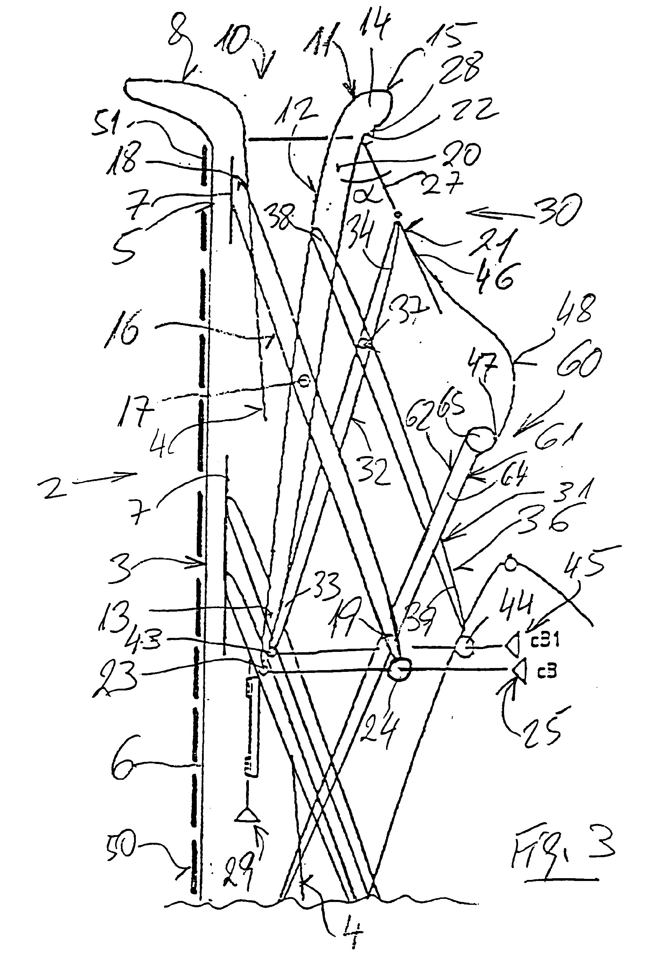

Fig. 2 zeigt in einer Seitenansicht eine der Ausführungsformen der vorliegenden Einrichtung. Fig. 3 zeigt einen vergrösserten Ausschnitt aus dem oberen Bereich von Fig. 2. Die vorliegende Einrichtung ist grösstenteils anhand eines sogenannten kinematischen Schemas dargestellt. Dieses Schema zeigt unter anderem auch das Prinzip der vorliegenden Einrichtung für einen bestimmten Bereich des Beines. Dieses Prinzip lässt sich dann in einer Einrichtung dieser Art wiederholt anwenden, um mehrere Bereiche einer Beinbekleidung zu prüfen.

Die vorliegende Einrichtung weist eine Grundplatte 1 auf, auf welcher sich eine Hauptstütze 2 der vorliegenden Einrichtung befindet. Diese Hauptstütze 2 hat einen länglichen Grundkörper 3, welcher im wesentlichen als eine hohle Schiene ausgeführt ist. Diese Schiene 3 hat im in Fig. 2 bzw. 3 dargestellten Fall einen etwa U-förmigen Querschnitt. Diese Schiene 3 kann jedoch auch einen L-förmigen oder sogar einen viereckförmigen Querschnitt aufweisen. Die in der Grundplatte 1 eingesteckte Hauptstütze 2 ist kein zwingender Bestandteil der vorliegenden Einrichtung. Wichtig ist vor allem die Schiene 3, welcher die übrigen Bestandteile der vorliegenden Einrichtung zugeordnet sind. In Fig. 2 und 3 ist nur der vorne liegende Schenkel 4 der Schiene 3 mit dem U-förmigen Querschnitt dargestellt. Dieser vorne liegende U-Schenkel 4 ist über einen Verbindungssteg 5 mit dem zweiten U-Schenkel verbunden, welcher hinter dem vorderen U-Schenkel 4 liegt und durch diesen daher verdeckt ist. Zumindest die Aussenfläche 6 des Verbindungssteges 5 ist abgerundet. Es kann zweckmässig sein, auch die Innenfläche 7 des Verbindungssteges 5 entsprechend der abgerundeten Aussenfläche 6 desselben hohl auszuführen.

Die obere Endpartie der Hauptstütze 2 bzw. der Schiene 3 weist einen seitlich abgebogenen Abschnitt 8 auf. Dieser Schienenabschnitt 8 ist so abgebogen, dass er oberhalb des Verbindungssteges 5 der Schiene 3 liegt und sich zur Längsrichtung dieser Schiene 3 schräg aufwärts erstreckt. Dieser im wesentlichen hakenförmige Abschnitt 8 der Hauptstütze 2 simuliert den Bereich der Spitze eines Fusses. Bei einer weiteren Ausführungsform der vorliegenden Einrichtung kann der Bereich der Fussspitze 8 als ein Abschnitt eines schräg aufwärts verlaufenden, hohlen Formstückes, beispielsweise aus einem Kunststoff ausgeführt sein. Dieses Formstück ist den übrigen und nachstehend beschriebenen Bestandteilen dieser Einrichtung vorangestellt. Der Querschnitt dieses Formstückes kann bogenförmig verlaufen, und zwar etwa so, wie dies vorstehend im Zusammenhang mit dem Querschnitt der Hauptstütze 3 dargelegt ist.

Die vorliegende Einrichtung umfast ferner Messvorrichtungen 10. Die jeweilige Messvorrichtung 10 umfasst einen Hebelmechanismus und wenigstens einen Drucksensor 51. Die sich an der Messung aktiv beteiligenden Bestandteile der jeweiligen Messvorrichtung 10 befinden sich jeweils auf jenem Niveau L einer Beinbekleidung, wo Druckmessungen durchgeführt werden sollen. Zu diesem Zweck sind zumindest die Wirkflächen der Drucksensoren 51 an der Aussenfläche 6 der Schiene 3 angebracht. Deswegen kann die Schiene 3 auch als Träger der Drucksensoren 51 genannt werden. Die Drucksensoren 51 sind zu zumindest einer vertikal verlaufenden Sensorvorrichtung 50 zusammengefasst, wobei die Drucksensoren 51 innerhalb einer Sensorvorrichtung 50 eine Reihe bilden. Im in Fig. 2 und 3 dargestellten Fall befindet sich die Sensorvorrichtung 50 vorne in der Mitte der Frontfläche 5 des Trägers 3. Zweckmässiger ist es jedoch, wenn es zwei Sensorvorrichtungen 50 gibt, von welchen je eine einer der Flanken bzw. einem der Schenkel 4 des Trägers 3 zugeordnet ist. Die Wirkflächen der Drucksensoren 51 der Sensorvorrichtungen 50 befinden sich an der Aussenfläche der Schenkel 4 des Trägers 3. Jeder der Drucksensoren 51 ist einzeln an eine zentrale Auswerteeinheit (nicht dargestellt) elektrisch angeschlossen.

Im oberen Bereich von Fig. 2 ist neben anderem auch eine erste der genannten Messvorrichtungen 10 dargestellt, welche zur Messung des Druckes im Bereich cB der Fessel bestimmt und ausgeführt ist. Dieser Bereich der vorliegenden Einrichtung ist in Fig. 3 vergrössert dargestellt. Die Messvorrichtung 10 umfasst neben anderem auch einen Hebelmechanismus 11 (Fig. 3), welcher einen ersten Hebel 12 aufweist. Dieser Hebel 12 erstreckt sich im wesentlichen vertikal. Das unten liegende Ende 13 dieses ersten Hebels 12 ist im dargestellten Beispiel an der Hauptstütze 2 unterhalb der Abkröpfung 8 mittels einer Achse 23 schwenkbar gelagert. Zweckmässigerweise ist dieses untere Ende 13 des ersten Hebels 12 am in Fig. 2 bzw. 3 im Vordergrund liegenden U-Schenkel 4 des Stützengrundkörpers 3 schwenkbar gelagert. Das obere Ende 14 des ersten Hebels 12 befindet sich etwa auf der Höhe des hakenförmigen Ausläufers 8 des Stützengrundkörpers 3. Das obere Ende 14 des ersten Hebels 12 weist ebenfalls einen seitlich abgebogenen Ausläufer 15 auf, welcher allerdings so abgebogen ist, dass er in einer zur Richtung der Abkröpfung 8 am Stützengrundkörper 3 entgegengesetzten Richtung zeigt. Der Ausläufer bzw. die Abkröpfung 15 am oberen Ende 14 des ersten Hebels 12 simuliert die Ferse eines Fusses bzw. einer Beinbekleidung.

Der Hebelmechanismus 11 weist ferner einen zweiten Hebel 16 auf, welcher sich ebenfalls im wesentlichen vertikal erstreckt. Dieser zweite Hebel 16 kreuzt den ersten Hebel 12. Diese Hebel 12 und 16 sind im Bereich ihrer Kreuzungsstelle mit Hilfe einer Achse 17 miteinander schwenkbar verbunden. Diese sich so zugeordneten Hebel 12 und 16 bilden einen sogenannten Scherenmechanismus. Die genannte Achse 17 liegt im dargestellten Fall etwa in der Mitte der Länge der genannten Hebel 12 und 16. Das oben liegende Ende 18 des zweiten Hebels 16 dieses Scherenmechanismus 11 liegt im dargestellten Beispiel auf der Innenfläche 7 des Stützengrundkörpers 3 auf.

Das unten liegende Ende 19 des zweiten Hebels 16 ist an die Spindelmutter 24 eines Spindelantriebs 25 für den Wert cB angeschlossen. Dieser Spindelantrieb 25 ist horizontal orientiert und er ist am Stützengrundkörper 3 etwa dort angebracht, wo das untere Ende 13 des ersten Hebels 12 schwenkbar gelagert ist. Der Spindelantrieb 25 ist so angeordnet und ausgeführt, dass er die unteren Enden 13 und 19 der Hebel 12 und 16 des Scherenmechanismus 11 gegeneinander zu und voneinander weg bewegen kann. Dementsprechend wird der Abstand zwischen den Abkröpfungen 8 und 15 an den oberen Enden dieser Hebel 12 und 16 vermindert oder vergrössert.

Eine Stange 21 ist mittels eines Gelenks 22 am oberen Schenkel 20 des ersten Hebels 12 einerends schwenkbar angebracht. Dieses Gelenk 22 befindet sich unterhalb der Abkröpfung 15 des ersten Hebels 12, sodass dieses Gelenk 22 an der von den Drucksensoren 51 abgewandten Seite des oberhalb der Schwenkachse 17 liegenden Schenkels 20 des ersten Hebels 12 liegt. Das Gelenk 22 befindet sich in einem Abstand 28 unterhalb der Abkröpfung 15 am oberen Ende 14 des ersten Hebels 12. Innerhalb dieses Abschnittes 28 des Hebels 12, welcher sich zwischen der Abkröpfung 15 und dem Gelenk 22 erstreckt, liegt jenes Niveau der vorliegenden Einrichtung, wo die Messung des Wertes cB durchgeführt wird, und zwar unter Zuhilfenahme des zuoberst liegenden Drucksensors 51. Weil der genannte Abschnitt 28 einen festen Bestandteil des ersten Hebels 12 darstellt, kann der Abstand zwischen vom Sensor 51 und der von diesem abgewandt liegenden Fläche 28 am Hebel 12 mit Hilfe des genannten ersten Spindelantriebs 25 ebenfalls verstellt werden.

Damit die Lage der Ferse 15 und somit auch des Messniveaus cB in einer vertikalen Richtung geändert werden kann, womit die Länge des Beines während der Herstellung der Beinbekleidung berücksichtigt werden kann, ist das Schwenklager 23 des unteren Endes 13 des ersten Hebels 12 an der Spindelmutter eines vertikal orientierten und fest möntierten Spindelantriebs 29 befestigt.

Im oberen Bereich von Fig. 2 sind ferner aktive Teile einer zweiten Messvorrichtung 30 dargestellt, welche zur Messung des Druckes im Bereich cB1 des Anfangs der Wade bestimmt und ausgeführt ist. Diese Messvorrichtung 30 umfasst unter anderem auch einen Hebelmechanismus 31, welcher einen ersten Hebel 32 aufweist. Dieser Hebel 32 erstreckt sich im wesentlichen vertikal. Das unten liegende Ende 33 dieses ersten Hebels 32 ist schwenkbar gelagert. Zweckmässigerweise ist dieses Ende 13 des ersten Hebels 32 am in Fig. 2 bzw. 3 vorne liegenden U-Schenkel 4 des Stützengrundkörpers 3 mittels einer Welle 43 schwenkbar gelagert. Das obere Ende 34 des ersten Hebels 32 befindet sich in einem Abstand unterhalb der Abkröpfung 15 am ersten Hebel 12 und es ist der Stange 27 zugeordnet. In dieser Stange 27 ist ein Schlitz (nicht dargestellt) ausgeführt, welcher sich zwischen den Enden dieser Stange erstreckt. Dieser Schlitz beginnt und endet in einem Abstand von den Enden dieser Stange 21. In diesem Schlitz ist das obere Ende 34 des ersten Hebels 32 der zweiten Messvorrichtung 30 längsverschiebbar gelagert.

Der zweite Hebelmechanismus 31 weist ferner einen zweiten Hebel 36 auf, welcher sich ebenfalls im wesentlichen vertikal erstreckt. Dieser zweite Hebel 36 kreuzt den ersten Hebel 32. Diese Hebel 32 und 36 sind im Bereich ihrer Kreuzungsstelle mit Hilfe einer Achse 37 miteinander schwenkbar verbunden. Diese sich so zugeordneten Hebel 32 und 36 bilden somit einen zweiten Scherenmechanismus. Die genannte Achse 37 liegt im dargestellten Fall oberhalb der Mitte der Länge der genannten Hebel 32 und 36. Das oben liegende Ende 38 des zweiten Hebels 36 dieses Scherenmechanismus 31 stützt sich im dargestellten Fall am oberen Schenkel 20 des ersten Hebels 12 des ersten Scherenmechanismus 11 ab. Unter Umständen wäre es möglich, dass das oben liegende Ende 38 des zweiten Hebels 36 dieses zweiten Scherenmechanismus 31 auf der Innenfläche 7 des Stützengrundkörpers 3 aufliegt.

Das unten liegende Ende 39 des zweiten Hebels 36 ist an die Spindelmutter 44 eines zweiten Spindelantriebs 45 für den Wert cB1 angeschlossen. Dieser Spindelantrieb 45 ist horizontal orientiert und er ist etwa dort angebracht, wo das untere Ende 33 des ersten Hebels 32 mittels der Welle 43 schwenkbar gelagert ist. Der Spindelantrieb 45 ist so angeordnet und ausgeführt, dass er die unteren Enden 33 und 39 der Hebel 32 und 36 des zweiten Scherenmechanismus 31 gegeneinander zu und voneinander weg bewegen kann.

Dementsprechend wird der Abstand zwischen den oberen Enden 34 und 38 der Hebel 32 und 36 vermindert oder vergrössert. Wenn der Abstand zwischen den oberen Enden 34 und 38 der Hebel 32 und 36 geändert wird, dann bewegt sich das obere Ende 34 des ersten Hebels 32 nach rechts. Da dieses Ende 34 des ersten Hebels 32 in der Stange 21 gelagert ist, vergrössert sich dabei ein Winkel Alpha, welcher sich zwischen dem oberen Schenkel 20 des ersten Hebels 12 und der Stange 21 erstreckt. Dadurch ändert sich auch der Abstand zwischen der Rückseite der Stange 21 und dem auf demselben Niveau liegenden Drucksensor 51. In dieser Weise können unterschiedliche Grössen des Umfanges des Beines im Bereich cB1 des Anfangs der Wade eingestellt werden.

Damit die Lage des Messbereichs cB1 in einer vertikalen Richtung geändert werden kann, womit die Länge des Beines eines Patienten berücksichtigt werden kann, kann die Lagerwelle 43 des ersten Hebels 32 an der Spindelmutter des bereits genannten vertikal orientierten Linearantriebs 29 (Fig. 2) gelagert sein.

Im oberen Bereich von Fig. 2 sind aktive Teile auch einer dritten Messvorrichtung 60 dargestellt, welche zur Messung des Druckes im Bereich cC der Mitte der Wade bestimmt und ausgeführt ist. Diese Messvorrichtung 60 umfasst unter anderem auch einen Hebelmechanismus 61, welcher einen ersten Hebel 62 aufweist. Dieser Hebel 62 erstreckt sich im wesentlichen vertikal. Das unten liegende Ende 63 dieses ersten Hebels 62 ist schwenkbar gelagert. Zweckmässigerweise ist dieses Ende 63 des ersten Hebels 62 mittels einer Welle 53 schwenkbar gelagert. Dem oberen Ende 64 des ersten Hebels 62 ist ein längliches Formstück 48 zugeordnet. Das oben liegende Ende 46 dieses Formstücks 48 ist der Stange 21 gleitbar zugeordnet. Das unten liegende Ende 47 des Formstückes 48 ist über ein Gelenk 65 am oben liegende Ende 64 des ersten Hebels 62 schwenkbar gelagert. Das Formstück 48 ist so ausgeführt, dass seine von den Drucksensoren 51 abgewandte Seite eine Oberfläche aufweist, welche der Oberfläche der Rückseite einer Wade ähnelt.

Dieser dritte Hebelmechanismus 61 weist ferner einen zweiten Hebel 66 auf, welcher sich ebenfalls im wesentlichen vertikal erstreckt. Dieser zweite Hebel 66 kreuzt den ersten Hebel 62. Diese Hebel 62 und 66 sind im Bereich ihrer Kreuzungsstelle mit Hilfe einer Achse 67 miteinander schwenkbar verbunden. Diese sich so zugeordneten Hebel 62 und 66 bilden somit einen dritten Scherenmechanismus. Das oben liegende Ende 68 des zweiten Hebels 66 dieses Scherenmechanismus 61 ist gleitbar abgestützt. Das unten liegende Ende 69 des zweiten Hebels 66 ist an die Spindelmutter 54 eines dritten Spindelantriebs 55 für den Wert cC angeschlossen. Dieser Spindelantrieb 55 ist horizontal orientiert und er ist etwa dort angebracht, wo das untere Ende 63 des ersten Hebels 62 mittels der Welle 53 schwenkbar gelagert ist.

Der Spindelantrieb 55 ist so angeordnet und ausgeführt, dass er die unteren Enden 63 und 99 der Hebel 62 und 66 des dritten Scherenmechanismus 61 gegeneinander zu und voneinander weg bewegen kann. Dementsprechend wird der Abstand zwischen den oberen Enden 64 und 68 der Hebel 62 und 66 vermindert oder vergrössert. Wenn der Abstand zwischen den oberen Enden 64 und 68 der Hebel 62 und 66 vergrössert wird, dann bewegt sich das obere Ende 64 des ersten Hebels 62 nach rechts. Da das untere Ende 47 des Formstückes 48 an das obere Ende 64 des ersten Hebels 62 angeschlossen ist, bewegt sich das untere Ende 47 des Formstückes 48 ebenfalls nach rechts, womit sich der Abstand zwischen der rückwärtigen Oberfläche am Formstück 48 und den auf demselben Niveau liegenden Drucksensoren 51 vergrössert. In dieser Weise können unterschiedliche Grössen des Umfanges des Beines im Bereich cC der Wade in der vorliegenden Einrichtung eingestellt werden.

Damit die Lage des Messbereichs LC in einer vertikalen Richtung geändert werden kann, womit die Länge des Beines eines Patienten berücksichtigt werden kann, ist die Lagerwelle 63 des ersten Hebels 62 an einem zweiten vertikal orientierten Linearlager 49 angebracht.

In Fig. 2 ist noch eine vierte Messvorrichtung 70 dargestellt, mit welcher die Abmessungen im Bereich eines menschlichen Oberschenkels eingestellt werden können und mit welcher die Messungen von Druck zwischen den Bereichen cD und cG (Fig. 1) durchgeführt werden können. Diese Messvorrichtung 70 umfasst unter anderem auch ein zweites längliches Formstück 88. Die von den Drucksensoren 51 abgewandt liegende Oberfläche 89 des Formstückes 88 hat einen Verlauf, welcher dem Verlauf der Oberfläche der rückwärtigen Seite des Oberschenkels entspricht. Das untere Ende 87 dieses Formstückes 88 ist an die Mutter eines weiteren horizontal orientierten Spindelantriebs 84 angeschlossen, welcher die Einstellung des Werte cG (Fig. 1) im oberen Bereich LG des Oberschenkels ermöglicht.

Diese Messvorrichtung 70 umfasst ferner einen vierten Hebelmechanismus 71, welcher einen ersten Hebel 72 aufweist. Dieser Hebel 72 erstreckt sich im wesentlichen vertikal. Das unten liegende Ende 73 dieses ersten Hebels 72 ist schwenkbar gelagert. Zweckmässigerweise ist dieses Ende 73 des ersten Hebels 72 mittels einer Welle 85 schwenkbar gelagert. Dem oberen Ende 74 des ersten Hebels 72 ist das obere Ende 86 des zweiten länglichen Formstückes 88 schwenkbar zugeordnet.

Dieser vierte Hebelmechanismus 71 weist ferner einen zweiten Hebel 76 auf, welcher sich ebenfalls im wesentlichen vertikal erstreckt. Dieser zweite Hebel 76 kreuzt den ersten Hebel 72. Diese Hebel 72 und 76 sind im Bereich ihrer Kreuzungsstelle mit Hilfe einer Achse 77 miteinander schwenkbar verbunden. Diese sich so zugeordneten Hebel 72 und 76 bilden somit einen vierten Scherenmechanismus. Das oben liegende Ende 78 des zweiten Hebels 76 dieses Scherenmechanismus 71 ist gleitbar abgestützt. Das unten liegende Ende 79 des zweiten Hebels 76 ist an die Spindelmutter eines weiteren Spindelantriebs 95 für die Einstellung des Wertes cD angeschlossen. Dieser Spindelantrieb 95 ist horizontal orientiert und er ist etwa dort angebracht, wo das untere Ende 73 des ersten Hebels 72 mittels der Welle 85 schwenkbar gelagert ist.

Der Spindelantrieb 95 ist so angeordnet und ausgeführt, dass er die unteren Enden 73 und 79 der Hebel 72 und 76 dieses Scherenmechanismus 71 gegeneinander zu und voneinander weg bewegen kann. Dementsprechend wird der Abstand zwischen den oberen Enden 74 und 78 der Hebel 72 und 76 vermindert oder vergrössert. Wenn der Abstand zwischen den oberen Enden 74 und 78 der Hebel 72 und 76 vergrössert wird, dann bewegt sich das obere Ende 74 des ersten Hebels 72 nach rechts. Das obere Ende 86 des Formstückes 88 ist an das obere Ende 74 des ersten Hebels 72 angeschlossen. Deswegen bewegt sich das obere Ende 86 des Formstückes 88 ebenfalls nach rechts, womit sich der Abstand zwischen der rückwärtigen Oberfläche am Formstück 88 und den auf demselben Niveau liegenden Drucksensoren 51 vergrössert. In dieser Weise können unterschiedliche Grössen des Umfanges des Beines im unteren Bereich LD des Oberschenkels in der vorliegenden Einrichtung eingestellt werden.

Damit die Lage LG der Messbereiche cG und cD in einer vertikalen Richtung geändert werden kann, womit die Länge des Beines eines Patienten berücksichtigt werden kann, sind die Lagerwelle 85 des ersten Hebels 72 und der Spindelantrieb 84 an einem weiteren vertikal orientierten Linearlager 99 angebracht.

Die Hebel aller Scherenmechanismen können als Flacheisen ausgeführt sein, sodass der jeweilige Scherenmechanismus sehr flach ausfällt. Folglich nehmen mehrere sich gegenseitig flach zugeordnete Scherenmechanismen ein nur geringes Volumen ein, sodass auch eine grössere Anzahl solcher Scherenmechanismen im Inneren einer Beinbekleidung 80 Platz finden können.

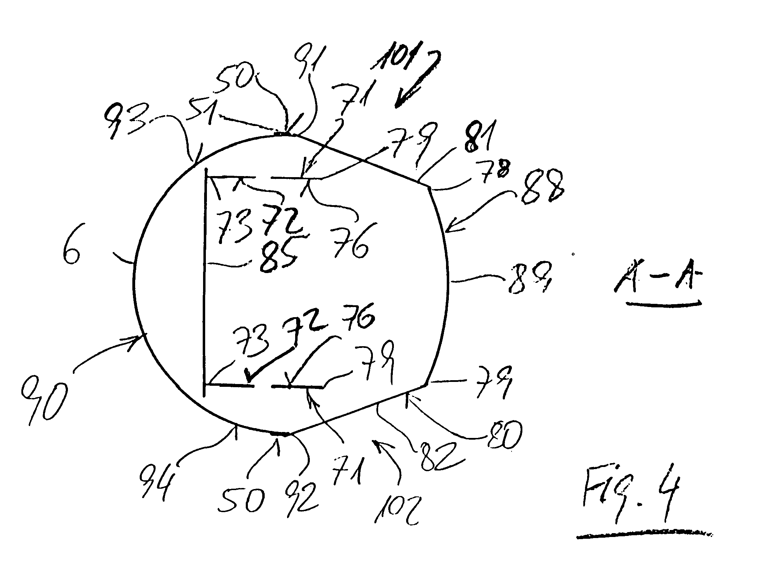

Fig. 4 zeigt einen bloss schematischen horizontalen Schnitt A-A durch eine weitere Ausführung der vorliegenden Erfindung, welche der Einrichtung gemäss Fig. 2 entspricht. Die Einrichtung gemäss Fig. 4 weist den länglichen und vertikal verlaufenden Träger 90 für die Drucksensoren 51 auf. Dieser Träger 90 ist den vorstehend beschriebenen Hebelmechanismen 10 vorangestellt ist. Die obere Endpartie dieses Trägers 90 kann die vorstehend genannte Abkröpfung 8 (Fig. 2) aufweisen, welche die Spitze eines Fusses simuliert. Der Querschnitt dieses Trägers 90 ist bogenförmig, wobei der Zentriwinkel dieses Bogens zweckmässigerweise 180 Grad aufweist. Ein solcher Träger 90 weist zwei Flanken 93 und 94 mit zwei sich vertikal erstreckende Randpartien 91 und 92 auf. Dieser Träger 90 simuliert in der vorliegenden Einrichtung für die Beinbekleidung den Bereich des Schienbeines.

Die gekrümmte Fläche 89 am Formstück bzw. Gegenlager 88, das in Fig. 4 dargestellt ist, simuliert in der vorliegenden Einrichtung dagegen die rückwärtige Oberfläche des Oberschenkels. Bei dieser Ausführungsform der vorliegenden Einrichtung sind zwei Gruppen 50 von Drucksensoren 51 vorgesehen, wobei die Drucksensoren 51 innerhalb der jeweiligen Gruppe eine praktisch vertikal verlaufende Reihe bilden. Je eine dieser Reihen 50 von Drucksensoren 51 ist der Aussenseite einer der Flanken 93 bzw. 94 oder der Aussenseite einer der Randpartien 91 bzw. 92 des Trägers 90 zugeordnet. Der Träger 90 samt den Sensorreihen 50 und das Formstück 88 sind von der Beinbekleidung 80 umgeben. Zwischen den Vertikalkanten 91 und 92 des Trägers 90 und den Vertikalkanten 78 und 79 des Formstückes 88 erstrecken sich die dazwischen frei stehenden Abschnitte 81 und 82 der über diese Einrichtung überstülpten Beinbekleidung 80.

Zwischen den Flanken 93 und 94 des Trägers 90 mit dem bogenförmigen Querschnitt liegen die vorderen Abschnitte der genannten Hebelmechanismen 11, 31, 61 und 71. Aus dem in Fig. 4 dargestellten Schnitt ist auch ersichtlich, dass diese Ausführung der vorliegenden Einrichtung zwei Anordnungen 101 und 102 aufweist, von welchen jede die vorstehend beschriebenen Hebelmechanismen 11, 31, 61 und 71 aufweist. Jede dieser Anordnungen 101 und 102 ist im wesentlichen flach, weil die Hebel der Hebelmechanismen 11, 31, 61 und 71 aus Flacheisen sind. Solche flache Anordnungen 101 und 102 verlaufen vertikal und sie befinden sich in einem horizontalen Abstand voneinander, sodass sie parallel zueinander angeordnet sind. Die Gegenlager bzw. Formstükke 21, 28, 48 und 88 erstrecken sich zwischen den Anordnungen 101 und 102. Die vorstehend beschriebenen Schwenkwellen sind für die beiden Anordnungen 101 und 102 gemeinsam. Diese Situation ist in Fig. 4 beispielsweise durch die Schwenkwelle 85 dargestellt, welche sich zwischen den unten liegenden Enden 73 der ersten Hebel 72 der vierten Hebelvorrichtung 70 der nebeneinander angeordneten und vertikal verlaufenden Anordnungen 101 und 102 erstreckt.

Aus Fig. 4 sind auch die unten liegenden Enden 79 der zweiten Hebel 76 der vierten Hebelvorrichtung 70 ersichtlich, welche an einen gemeinsamen Spindelantrieb 95 (in Fig. 4 nicht dargestellt) angeschlossen sind. Auch die übrigen Spindelantriebe 25, 45 und 55 sowie die Linearlager 29, 49 und 99, welche im Zusammenhang mit Fig. 2 bzw. 3 vorstehend beschrieben worden sind, können für die beiden Anordnungen 101 und 102 gemeinsam sein. In diesem Fall befinden sich die Spindelantriebe 25, 45 und 55 und die Linearlager 29, 49 und 99 zwischen den Anordnungen 101 und 102 und die betreffenden Hebel der Anordnungen 101 und 102 sind an die einander gegenüberliegende Seiten der Spindelantriebe 25, 45 und 55 und der Linearlager 29, 49 und 99 gekoppelt. Die in Fig. 4 dargestellte Ausführung der vorliegenden Einrichtung kommt ohne die Hauptstütze 2 (Fig. 2 bzw. 3) aus. Dies deswegen, weil die parallel nebeneinander stehenden und horizontal untereinander gekoppelten Anordnungen 101 und 102 ein selbst tragendes Gebilde darstellen. Jene Enden der Hebel, welche bei der in Fig. 2 und 3 dargestellten Einrichtung an der Hauptstütze 2 abgestützt sind, sind an anderen Hebeln der jeweiligen Anordnung 101 bzw. 102 abgestützt, und zwar etwa in der Art, wie dies beim Ende 38 des Hebels 36 in Fig. 2 bzw. 3 der Fall ist.

Wenn eine hergestellte Beinbekleidung 80, z.B. ein Strumpf oder dgl. vermessen werden soll, dann wird diese über die vorliegende Einrichtung von oben her so gestülpt, dass der Fortsatz 8 im Inneren der Spitze des Strumpfes liegt und dass der Fortsatz 15 am oberen Ende 14 des ersten Hebels 12 im Inneren der Ferse des Strumpfes liegt. Der Schaft des Strumpfes 80 wird dann weiter tiefer über die übrigen Bestandteile der vorliegenden Einrichtung gezogen. Die Beinbekleidung 80 liegt jetzt vorne auf der Oberfläche 6 des Trägers 90 mit den Drucksensoren 50 und hinten auf der Oberfläche 89 des unteren Formstückes 88 und unter Umständen auch auf den Oberflächen der übrigen Formstücke bzw. Gegenlager auf. In Einrichtungen des Standes der Technik wurden Beinbekleidungen in einem flach gelegten Zustand vermessen, was der Situation beim Tragen der Beinbekleidung nicht gerecht war. Die Beinbekleidung 80 wird mittels der vorliegenden Einrichtung im offenen Zustand geprüft, d.h. im gleichen Zustand als wenn die Beinbekleidung auf einem Bein aufgezogen wäre. Die zu testende Beinbekleidung 80 umgibt dabei die Aussenflächen sowohl des Trägers 90 als auch der Gegenlager und die Innenseite der Beinbekleidung übt Druck auf die Drucksensoren 51 aus. Der Druck wird vorteilhaft durch die an den Flanken 93 und 94 des Trägers 90 angeordneten Sensoren 50 gemessen, was zu einer genaueren Erfassung der Grösse des Druckes in den vertikal übereinander angeordneten Niveaus bzw. Regionen LB, LB1, LC, LF, LG usw. der Beinbekleidung 80 beiträgt.

Die Wirkung c der Beinbekleidung 80 auf den Niveaus LB, LB1, LC, LF, LG usw. kann in der Weise gemessen werden, dass zuerst bestimmte Abstände zwischen dem Träger 90 und den aktiven und zum Träger 90 gegenüber liegenden mechanischen Teilen der einzelnen Spannvorrichtungen 10, 30, 60 und 70 eingestellt werden, nachdem eine Beinbekleidung 80 auf diese Einrichtung aufgezogen worden ist. Die genannten Abstände entsprechen Werten, welche jenen Werten gleichen, die während der Vermessung des Beins des Patienten auf den genannten Niveaus LB, LB1, LC, LF, LG usw. ermittelt worden sind. Hiernach werden die Druckwerte von jenen Drucksensoren 51 bzw. Drucksensorgruppen abgelesen, welche auf den einzelnen Niveaus LB, LB1, LC, LF, LG usw. liegen. Falls die abgelesene Druckwerte derart sind, dass sie die angestrebte gesundheitliche Wirkung und zugleich auch ein komfortables Tragen der Beinbekleidung 80 ermöglichen, so ist die Beinbekleidung entsprechend den Vorgaben hergestellt worden.

Damit man während der Vermessung von Beinbekleidungen rationell vorgehen kann, ist es zweckmässig, dass die Spindeln nicht nur der horizontal wirkenden Spindelantriebe sondern auch die Spindeln der vertikal wirkenden Linearlager durch elektrische Motoren, z.B. durch Schrittschaltmotoren angetrieben werden. Von der Auswertevorrichtung aus werden diese Motoren so angesteuert, dass die Abstände zwischen den Drucksensoren 51 und den auf dem jeweiligen Niveau LB, LB1, LC, LF, LG usw. gegenüber liegenden Wirkorganen der jeweiligen Messvorrichtung für den jeweiligen Bereich der Beinbekleidung schnell und genau eingestellt werden. Druckwerte, welche durch die auf den genannten Niveaus LB, LB1, LC, LF, LG usw. liegenden Drucksensoren 51 geliefert werden, werden mit den für das betreffende Niveau LB, LB1, LC, LF, LG usw. des Beines des Patienten geltenden Vorgaben verglichen. Aus dem Resultat dieses Vergleichs wird auf die Qualität der hergestellten Beinbekleidung geschlossen.

Die Ermittlung des Druckwertes auf dem jeweiligen Niveau LB, LB1, LC, LF, LG usw. basiert auf der Tatsache, dass die Drucksensoren 51 bzw. die Gruppen der Drucksensoren 51 der Druckmessvorrichtungen 50 an die Auswertevorrichtung einzeln angeschlossen sind. Zur Ermittlung der Grösse des Drukkes auf den während der Vermessung des Beines des Patienten ermittelten Niveaus LB, LB1, LC, LF, LG usw. werden jene Drucksensoren 51 bzw. Gruppen der Drucksensoren durch die Auswertevorrichtung abgefragt, welche auf den Niveaus LB, LB1, LC, LF, LG usw. liegen. So können die gesuchten Druckwerte schnell, genau und ohne zusätzliche mechanische Mittel festgestellt werden. Zur Durchführung einer solchen Auswertung von fertigen Produkten kann man sich eines Computerprogramms bedienen, welches aufgrund der während der Vermessung des Beines eines Patienten ermittelten Vorgaben die Abstände zwischen den Drucksensoren 51 und den auf dem jeweiligen Niveau LB, LB1, LC, LF, LG usw. liegenden Gegenlager in der jeweiligen Messvorrichtung für den jeweiligen Bereich des Beines automatisch einstellt, anschliessend die Druckwerte auf dem jeweiligen Niveau LB, LB1, LC, LF, LG usw. automatisch abliest und diese dann auswertet.

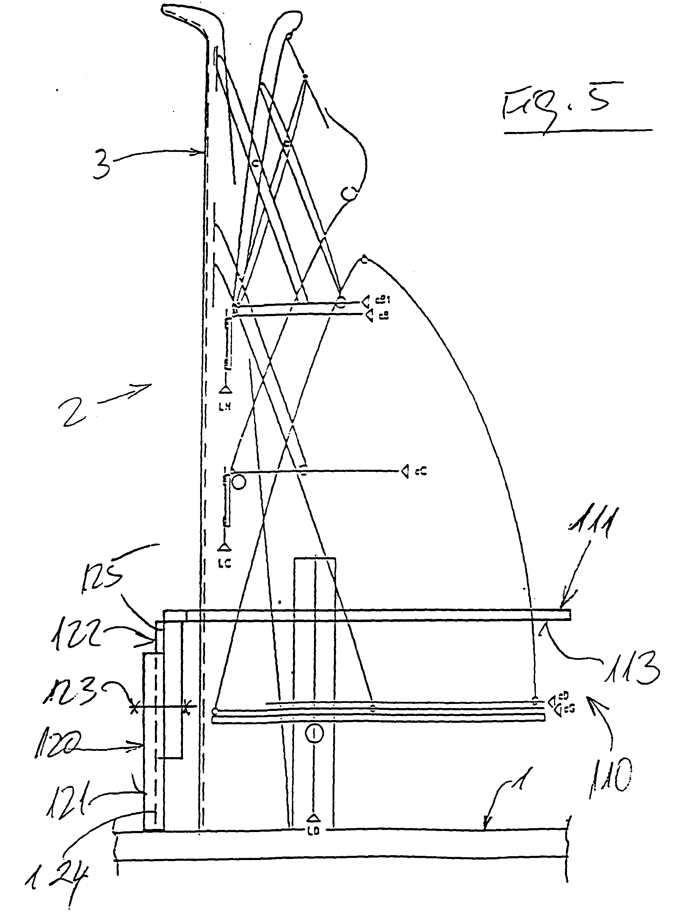

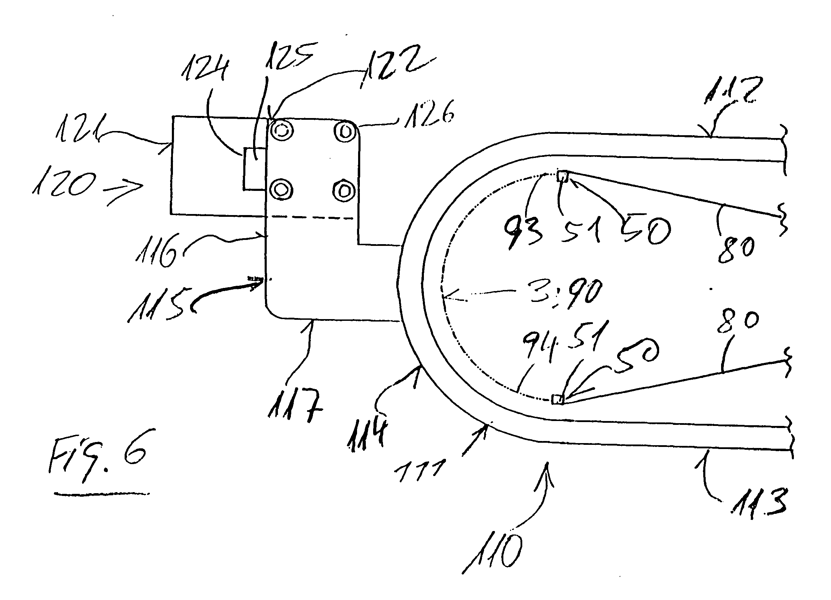

Fig. 5 zeigt eine noch weitere Ausführungsform der vorliegenden Einrichtung, deren Darstellung weitgehend der Darstellung in Fig. 2 entspricht. Fig. 6 zeigt in einer Draufsicht die in Fig. 5 dargestellte Ausführungsform der vorliegenden Einrichtung, nachdem die für diese Ausführungsform nicht wesentlichen Bestandteile der vorliegenden Einrichtung der besseren Übersicht wegen ausgelassen worden sind.

In jenem Bereich der Hauptstütze 2 (Fig. 5) der vorliegenden Einrichtung, wo sich die offene Endpartie der Beinbekleidung, hauptsächlich eines Strumpfes, während der Vermessung derselben befindet, ist eine Haltevorrichtung 110 für diese Endpartie der Beinbekleidung angeordnet. Dieser Bereich befindet sich oberhalb des unteren Endes des Trägers 3, welches in der Grundplatte 1 eingesteckt sein kann. Die Haltevorrichtung 110 umfasst einen Bügel 111, an welchen der Rand der offenen Endpartie der Beinbekleidung anschliessbar ist. Dieser Bügel 111 liegt in einer horizontalen Ebene und er ist U-förmig.

Der Bügel 111 weist zwei parallel zueinander verlaufende Schenkel 112 und 113 auf. Die einen Enden dieser Schenkel 112 und 113 sind mittels eines Stegs 114 zur genannten U-Form miteinander verbunden. Im dargestellten Fall ist der Steg 114 bogenförmig. An der Aussenseite dieses Steges 114 ist eine Anschlussplatte 115 einerends befestigt, welche ebenfalls in der genannten horizontalen Ebene zumindest im wesentlichen liegt. Im dargestellten Fall ist diese Anschlussplatte 115 L-förmig, sodass sie zwei Schenkel 116 und 117 aufweist. Die freie Endpartie eines der L-Schenkel 117 der Anschlussplatte 115 ist an den Steg 114 des Bügels 111 angeschlossen.

Die Haltevorrichtung 110 umfasst ferner einen Mechanismus 120, welcher zur Verstellung der Lage des Bügels 111 gegenüber dem Träger 3 in horizontaler Richtung ermöglicht. Der Verstellmechanismus 120 umfasst zwei vertikal verlaufende Stangen 121 und 122, welche praktisch parallel zueinander sind und welche einander so zugeordnet sind, dass eine der Stangen entlang der anderen Stange gleiten kann. Im dargestellten Fall weisen diese Stangen 121 und 122 einen viereckförmigen Querschnitt auf und sie sind sich über eine ihrer Seitenflächen einander zugeordnet. Es sind Mittel 123 vorgesehen, mit deren Hilfe die eingestellte Lage einer der Stangen gegenüber der anderen Stange festgelegt werden kann. Diese Mittel 123 können beispielsweise eine Schraube und eine Mutter umfassen. Der Bolzen der Schraube geht durch die beiden Stangen 121 und 122 hindurch. Die Lage der Stangen 121 und 122 zueinander kann in der Weise festgestellt werden, dass eine Mutter am anderen Ende des Schraubbolzens angezogen wird.

Damit sich die Stangen 121 und 122 nur parallel zueinander bewegen können, ist im dargestellten Fall eine Längsnut 124 in jener Seitenfläche einer der Stangen 121 ausgeführt, welcher die andere Stange 122 zugeordnet ist. Die entsprechende Seitenfläche dieser anderen Stange 122 ist mit einem hervorstehenden Kamm 125 versehen, welcher von dieser Seitenwand der Stange 122 absteht und welcher in der Nut 124 längsverschiebbar gelagert ist.

Die untere Endpartie der ersten bzw. unteren Stange 121 ist auf oder in der Grundplatte 1 befestigt. An die obere Endpartie der zweiten bzw. oberen Stange 122 ist der Bügel 111 angeschlossen. Zu diesem Zweck ist die freie Endpartie des zweiten L-Schenkels 116 der Anschlussplatte 115 am Bügel 111 an das obere Ende der zweiten Stange 122 angeschlossen. In der Stirnfläche dieses Endes der zweiten Stange 122 sind Schraublöcher ausgeführt. In der freie Endpartie des zweiten L-Schenkels 116 der Anschlussplatte 115 sind den Schraublöchern entsprechende Öffnungen 126 ausgeführt. Mittel Schrauben (nicht dargestellt), welche durch diese Öffnungen 126 hindurchgehen, kann die freie Endpartie des zweiten L-Schenkels 116 auf der Stirnfläche der zweiten Stange befestigt sein.

Bei dieser Ausführungsform der vorliegenden Einrichtung sind zwei Sensorvorrichtungen 50 vorgesehen, wobei die Drucksensoren 51 innerhalb der jeweiligen Gruppe eine praktisch vertikal verlaufende Reihe bilden. Je eine dieser Sensorreihen 50 ist einer der Flanken 93 bzw. 94 des Trägers 3 mit dem bogenförmigen Querschnitt zugeordnet. Die Wirkflächen der Sensoren 51 dieser Reihen 50 befinden sich an der Aussenseite der jeweiligen Flanke 93 bzw. 94 des Trägers 3. Der Träger 3 samt den Sensorreihen 50 sind von der Beinbekleidung 80 umgeben, von der in Fig. 6 nur der vordere Abschnitt derselben angedeutet ist.

## Patentansprüche

1. Einrichtung zum Testen von elastischen textilen Beinbekleidungen, insbesondere von Strümpfen oder Strumpfhosen, von medizinischen Kompressions-Strümpfen und Stützstrümpfen, **dadurch gekennzeichnet, dass** die Einrichtung Druck messende Vorrichtungen (10,30,60,70) aufweist, welche so ausgeführt sind, dass sie sich während dem Testen von elastischen textilen Beinbekleidungen im Inneren der jeweiligen Beinbekleidung befinden, und dass die Wirkelemente der Druck messenden Vorrichtungen entlang der zu testenden Beinbekleidung verteilt sind.

2. Einrichtung nach Patentanspruch 1, **dadurch gekennzeichnet, dass** die jeweilige Druck messende Vorrichtung (10,30,60,70) wenigstens einen Drucksensor (51), einen Hebelmechanismus (11,31,61,71) und ein Gegenlager (21,28,48,88) umfasst, dass das Gegenlager als ein Formstück ausgeführt sein kann, dass der Drucksensor (51) bzw. eine Gruppe von Drucksensoren (50) zu einer Seite des Hebelmechanismus (11,31,61,71) angeordnet sind, dass das Gegenlager (21,28,48,88) an der gegenüberliegenden Seite des Hebelmechanismus (11,31,61,71) angeordnet ist und dass der Drucksensor (51) bzw. eine Gruppe von Drucksensoren (50) und das dazu gegenüberliegende Gegenlager (21,28,48,88) die Wirkelemente einer der Messvorrichtungen (10,30,60,70) darstellen.

3. Einrichtung nach Patentanspruch 2, **dadurch gekennzeichnet, dass** die Drucksensoren (51) an einem Träger (90) angebracht sind, welcher einen bogenförmigen Querschnitt hat, sodass dieser Träger den Schienbeinbereich simuliert, und dass das Gegenlager (21,28,48,88) als ein Formstück ausgeführt ist, dass die von den Drucksensoren (51) abgewandte Oberfläche des Formstückes den Verlauf der Oberfläche des entsprechenden Beinabschnittes nachahmt und dass die Breite des Formstückes der Breite des Beines im betreffenden Bereich desselben entspricht.

4. Einrichtung nach Patentanspruch 2, **dadurch gekennzeichnet, dass** die Drucksensoren (51) bzw. die Gruppen von Drucksensoren zumindest eine Reihe bilden, welche sich in der Längsrichtung der Einrichtung erstreckt und dass die Drucksensoren (51) bzw. die Gruppen von Drucksensoren an eine Auswertevorrichtung einzeln angeschlossen sind, welche die empfangenen Druckwerte verarbeiten und anzeigen kann.

5. Einrichtung nach Patentanspruch 2, **dadurch gekennzeichnet, dass** der Hebelmechanismus (11,31,61,71) einen ersten Hebel (12) und einen zweiten Hebel (16) aufweist, welche sich kreuzen und im Bereich ihrer Kreuzungsstelle mittels einer Schwenkwelle (17) miteinander schwenkbar verbunden sind, sodass diese Hebel (12,16) einen Scherenmechanismus darstellen, dass jeweils eines der zu den beiden Seite der Schwenkwelle (17) liegenden Enden (13,18) abgestützt ist, dass das zweite der zu einer Seite der Schwenkwelle (17) liegenden Enden (14) eines der Hebel (12) zur Verstellung der Gegenlager (21,28,48,88) bestimmt und ausgeführt ist und dass das zweite der zur gegenüberliegenden Seite der Schwenkwelle (17) liegenden Enden (19) des anderen Hebels (16) an eine horizontal wirkende Verstellvorrichtung (25) angeschlossen ist, mit deren Hilfe sich der Anstand zwischen den gegenüberliegenden Enden (14,18) der Hebel (12,16) ändern lässt.

6. Einrichtung nach Patentanspruch 5, **dadurch gekennzeichnet, dass** eine Stange (21) an die von den Drucksensoren (51) abgewandte Seite des oben liegenden Schenkels (20) des ersten Hebels (12) des ersten Hebelmechanismus (11) mittels eines Gelenks (22) angeschlossen ist und dass diese Stange (21) mit dem oben liegenden Schenkel (34) eines zweiten Scherenmechanismus (31) so gekoppelt ist, dass die Neigung Alpha der Stange (21) gegenüber einer Horizontalen mittels des genannten Schenkels (34) des zweiten Scherenmechanismus (31) verstellbar ist.

7. Einrichtung nach Patentanspruch 6, **dadurch gekennzeichnet, dass** ein erstes längliches Formstück (48) vorgesehen ist, welches die Wade simuliert, dass das untere Ende dieses Formstückes (48) dem oberen Ende (64) des ersten Hebels (62) eines dritten Hebelmechanismus (61) mittels eines Gelenks (65) zugeordnet ist, dass der oben liegende Endbereich (46) dieses Formstücks (48) der Stange (21) gleitbar zugeordnet ist und dass die von den Drucksensoren (51) abgewandte Seite des Formstücks (48) eine Oberfläche aufweist, welche der Oberfläche der Rückseite einer Wade ähnelt.

8. Einrichtung nach Patentanspruch 6, **dadurch gekennzeichnet, dass** ein zweites längliches Formstück (88) vorgesehen ist, dass die von den Drucksensoren (51) abgewandt liegende Oberfläche (89) dieses Formstückes (88) einen Verlauf hat, welcher dem Verlauf der Oberfläche der rückwärtigen Seite des Oberschenkels entspricht, dass das untere Ende (87) dieses Formstückes (88) an die Mutter eines horizontal orientierten Spindelantriebs (84) angeschlossen ist, dass das oben liegende Ende (86) des Formstückes (88) an das obere Ende (74) des ersten Hebels (72) eines vierten Scherenmechanismus (70) mittels eines Gelenks zugeordnet ist.

9. Einrichtung nach Patentanspruch 5, **dadurch gekennzeichnet, dass** die Hebelmechanismen (11,31,61,71) an vertikal wirkende Verstellvorrichtungen (29,49,99) angeschlossen sind.

10. Einrichtung nach Patentanspruch 2, **dadurch gekennzeichnet, dass** zwei Anordnungen (101,102) vorgesehen sind, von welchen je eine alle vorstehend beschriebenen Hebelmechanismen (11,31,61,71) aufweist, dass jede dieser Anordnungen (101,102) im wesentlichen flach ist, dass solche Anordnungen (101,102) vertikal verlaufen und sich in einem horizontalen Abstand voneinander befinden, sodass sie parallel zueinander angeordnet sind, dass die Schwenkwellen (23,43,53,85) für die beiden Anordnungen (101,102) gemeinsam sind.

11. Einrichtung nach Patentanspruch 1, **dadurch gekennzeichnet, dass** in jenem Bereich der Einrichtung, wo sich die offene Endpartie der Beinbekleidung (80) während der Vermessung derselben befindet, eine Haltevorrichtung (110) für diese Endpartie der Beinbekleidung (80) angeordnet ist.

12. Einrichtung nach Patentanspruch 11, **dadurch gekennzeichnet, dass** die Haltevorrichtung (110) einen praktisch horizontal verlaufenden Bügel (111) umfasst, dass dieser Bügel so ausgeführt ist, dass der Rand der offenen Endpartie der Beinbekleidung (80) an diesen Bügel anschliessbar ist, und dass dieser Bügel (111) an einem Verstellmechanismus (120) angebracht ist, welcher so ausgeführt ist, dass die Lage des Bügels (111) gegenüber der Schiene (3) der Einrichtung in vertikaler Richtung verstellbar ist.

13. Einrichtung nach Patentanspruch 12, **dadurch gekennzeichnet, dass** der Bügel (111) etwa U-förmig ist, dass die Schiene (3) sowie die Messvorrichtungen (10) sich zwischen den Schenkeln (112,113) dieses U-Bügels befinden und dass der Bügel (111) über einen Steg (114), welcher sich zwischen den einen Enden der Bügelschenkel (112,113) erstreckt, an den Verstellmechanismus (120) angeschlossen ist.

14. Einrichtung nach Patentanspruch 13, **dadurch gekennzeichnet, dass** der Verstellmechanismus (120) zwei Stangen (121,122) umfasst, welche praktisch parallel zueinander verlaufen und welche einander so zugeordnet sind, dass eine der Stangen entlang der anderen Stange gleiten kann, und dass Mittel (123) vorgesehen sind, welche die Feststellung der eingestellten Lage einer der Stangen gegenüber der anderen Stange ermöglichen.
